# EUROPEAN PATENT APPLICATION

(11) **EP 2 963 417 A1**
(43) Date of publication of application: **06.01.2016**
(21) Application number: 15172230.3
(22) Date of filing: 16.06.2015
(51) Int. Cl.: G01N 33/38, G01N 25/72, G01N 21/88

(54) **OPTICAL ANALYSIS OF CONCRETE**

(30) Priority: 18.06.2014 FI 20144149 U
(71) Applicant: A-Insinöörit Suunnittelu Oy, 33210 Tampere (FI)
(72) Inventor: Tarri, Mikko, 33210 Tampere (FI); Linne, Stina, 33210 Tampere (FI); Tulenheimo, Risto, 33210 Tampere (FI); Lehtola, Kari, 33210 Tampere (FI); Lehtonen, Arttu, 33210 Tampere (FI)
(74) Representative: Tampereen Patenttitoimisto Oy

(57) **Abstract**

The present invention relates to a device for analyzing a sample (30). The device (1) comprises at least means (15) for imaging the sample, and means (14) for illuminating the sample (30). The device (1) also comprises means (3) for moving the sample (30) in at least one direction, and means (16) for analyzing imaging information of the sample, for determining at least one property of the sample (30). Said means (14) for illuminating the sample are configured to illuminate the sample (30) from at least two different directions at different times. Further, said means (15) for imaging the sample are configured to image the sample (30) when the sample is being illuminated from said at least two different directions of illumination. Further, the invention relates to a method for analyzing a sample (30).

## Description

### Field of the invention

The present invention relates to a device for analyzing a sample, the device comprising means for imaging the sample and means for illuminating the sample. The invention also relates to a method for analyzing a sample, the method comprising imaging the sample and illuminating the sample.

### Background of the invention

Samples are taken from structures in order to analyze the condition of a building and possible damage and/or changes in the properties thereof. Such samples are taken, for example, from concrete parts of buildings, for example by drilling through the structure, whereby for example a cylindrical concrete sample is obtained as the drilling result. The analysis of this concrete sample may reveal changes in concrete which have possibly taken place in time and which may affect the properties of the concrete. One such change is carbonation. Carbonation, *i.e.* a neutralization reaction, results from the exposure of concrete to carbon dioxide, whereby the alkalinity (pH) of capillary water in the concrete is reduced. This may cause that the reinforcement in the concrete begins to rust and lose its strength properties, because the chemical protection (passive film) of the steel provided by the concrete is lost, exposing the structure to oxygen and moisture conditions which are favourable for rust development. The aim of analyzing a concrete sample is to find out the depth of carbonation in a concrete structure. It is possible to try to find out the depth of carbonation by using certain chemicals which change their colour according to the acidity. Thus, colour changes that can be detected in a concrete sample can indicate the acidity in different parts of the concrete sample, and the depth of carbonation can be found out in this way.

Another property to be possibly analyzed from a concrete sample is its porosity which may affect e.g. the strength of the concrete. Typically, a large number and/or size of pores may cause a reduction in the strength of the concrete compared to concrete with fewer pores and/or pores which are smaller in size. Moreover, porosity can also affect the way how easily carbon dioxide, water and other similar substances can penetrate the concrete structure.

Typically, the above mentioned properties have been analyzed visually by looking at the sample and evaluating e.g. the depth of carbonation and the number and size of pores in the sample. However, the reliability of such an analysis is largely based on the skills and experience of the analyzing person, which could mean that the analysis results of even the same sample are different when given by different persons.

Also, systems based on cameras have been developed, whereby a camera is used to take pictures of a sample, and properties of the sample are determined by looking at these pictures. This, too, is primarily based on visual analysis by a person, and the method may thus be unreliable to some extent.

### Brief summary of the invention

It is an aim of the present invention to provide a device for analyzing samples, whereby the analysis is largely based on processing information contained in images taken from a sample, by software. By means of the device, the sample can be illuminated from different directions to make the details in the surface structure more clearly visible, wherein by imaging different parts of the sample it is possible to produce one or more images, or a series of images, to be analyzed by software.

To put it more precisely, the device according to the present invention is primarily characterized in that the device comprises means for moving the sample at least around its longitudinal axis, and means for imaging the sample in connection with moving the sample. The method according to the present invention is primarily characterized in that in the method, the sample is further moved in at least one direction, and information contained in images taken from the sample is analyzed to determine at least one property of the sample, wherein in the method, the sample is illuminated from at least two different directions at different times, and the sample is imaged when it is being illuminated from said at least two different illumination directions.

### Description of the drawings

In the following, the present invention will be described in more detail with reference to the appended drawings, in which
- Fig. 1: shows a device according to an advantageous embodiment of the invention in a reduced view,
- Fig. 2: shows the device according to Fig. 1 in a side view,
- Figs. 3a to 3c: show reduced views of some advantageous arrangements of power transmission for moving a sample,
- Fig. 4: shows examples of forming shadows on the surface of a sample under different illuminations, and
- Fig. 5: shows some examples of samples that can be analyzed by the device.

### Detailed description of the invention

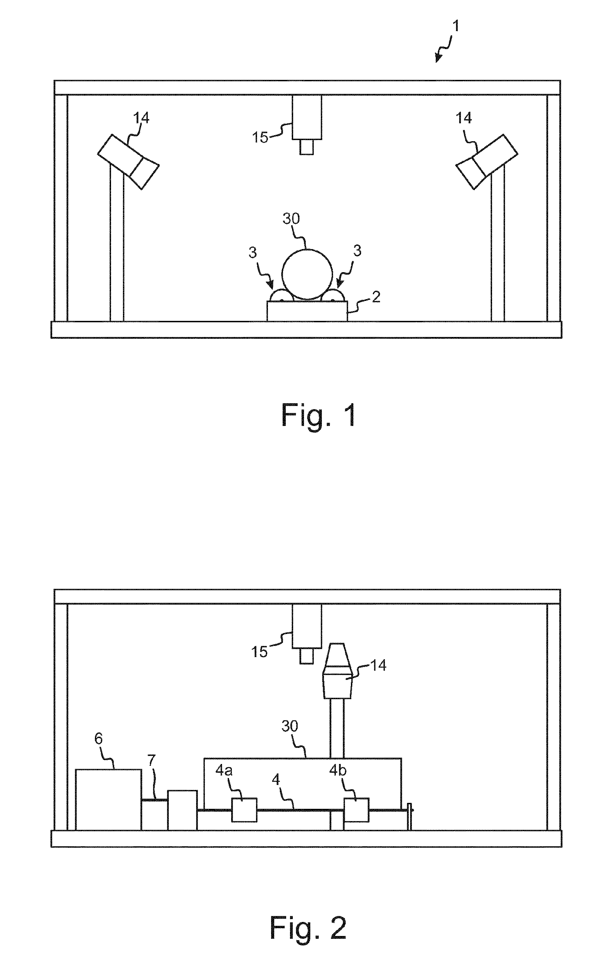

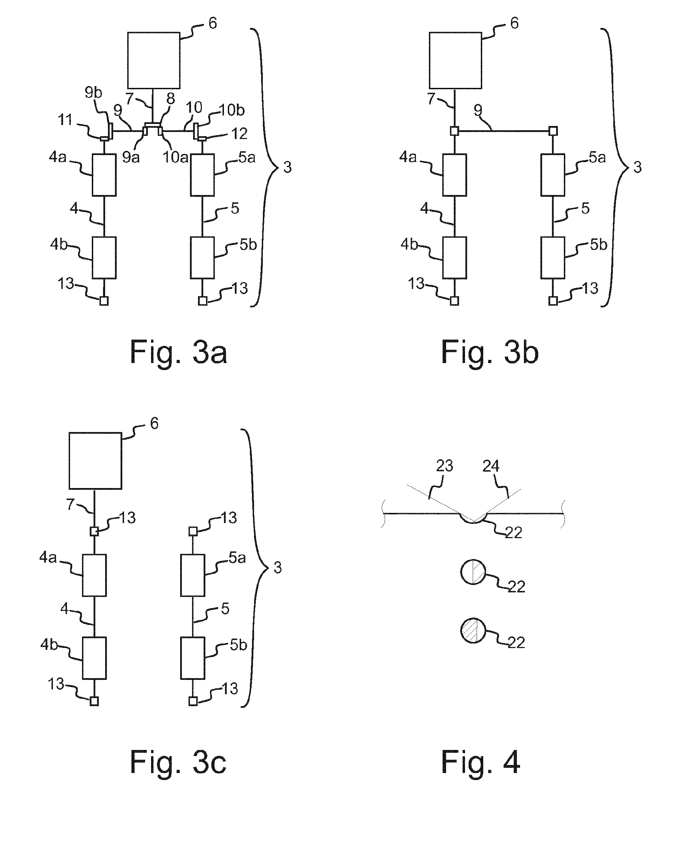

Figure 1 shows, in a reduced view, the principle of operation of a device 1 according to an advantageous embodiment of the invention. The device 1 comprises a handling support 2 for a sample, on which support a sample 30 can be placed for analysis. The handling support 2 comprises means 3 for moving the sample in at least one direction, advantageously for rotating the sample 30 about its longitudinal axis. The sample 30 to be analyzed is, for example, a cylindrical piece, wherein the sample 30 can be rotated about its longitudinal axis. In this advantageous embodiment, the means 3 for moving the sample comprise two elongated shafts 4, 5 equipped with two rollers 4a, 4b; 5a, 5b (Figs. 2 and 3a-3c) or corresponding pieces with rotational symmetry. It is obvious that the number of these shafts 4, 5 can also be one or more than two, and that the number of rollers 4a, 4b; 5a, 5b on each shaft 4, 5 can be different from two, for example one or three or more. The rollers 4a, 4b; 5a, 5b can be, for example, rubber rollers or corresponding pieces of rotational symmetry.

The device 1 also comprises a motor 6 or the like for generating the effective power needed for moving the sample. This effective power can be transmitted to the shafts 4, 5 by means of, for example, cogwheels, a belt, or a corresponding means for transmitting power. In an advantageous embodiment, as illustrated in a reduced view in Fig. 3a, a first cogwheel 8 is mounted on a motor shaft 7 and fitted to mesh with second cogwheels 9a, 10a at respective ends of auxiliary shafts 9, 10. In a corresponding way, third cogwheels 9b, 10b are provided at respective other ends of the auxiliary shafts 9, 10 to transmit power to fourth cogwheels 11, 12 at respective ends of the shafts 4, 5. Thus, the rotary motion of the motor shaft 7 generates a rotary motion of the shafts 4, 5 in such a way that the sample 30 is rotated in a desired direction, at a desired speed. It is obvious that for the sample 30 to rotate in the above described way, both of the shafts have to rotate in the same direction. Also, the speed of rotation has to be the same, if the diameter of all the rollers 4a, 4b; 5a, 5b is the same. If the diameter of all the rollers 4a, 4b; 5a, 5b is not the same, the rotation speeds of the shafts have to be set preferably such that the circumferential speed of each roller is the same. Thus, between the circumferential surface of the sample and the circumferential surface of the roller, no speed difference can be formed which could wear the surface of the roller.

As mentioned above, the power transmission from the motor 6 to the shafts 4, 5 can also be implemented by means of e.g. belts. It is thus possible to use belt pulleys or the like (not shown) instead of the cogwheel 8 on the motor shaft 7 and the cogwheels 9a, 10a at one end of the shafts. In this case, the auxiliary shafts are not necessarily needed. Other possible transmission means, such as a combination of cogwheels and belts, can be applied as well.

Figure 3b illustrates, in a reduced view, a transmission arrangement in which the motor shaft 7 is also used as said first shaft 4 or is directly connected to the shaft 4. Thus, the cogwheels, belts or other transmission mechanisms are also used for transmitting power from the motor shaft 7 to the second shaft 5.

In another advantageous embodiment of the invention, the sample is supported directly by the shafts 4, 5, wherein there is no need for separate rollers 4a, 4b, 5a, 5b at all.

Also, the means 3 for moving the sample can be implemented in such a way that the power transmission from the motor 6 is only connected to one shaft (for example, the first shaft 4), whereby the second shaft 6 is arranged to be freely rotating. Thus, the rotating motion of the first shaft 4 will make the sample rotate, which, in turn, will make the second shaft rotate because of the rotating motion of the sample. It will thus not be necessary to adjust the rotation speeds of the different shafts, because the rotation speed of the freely rotating shaft will be determined by the circumferential speed of the sample 30. Figure 3c shows such an example in a reduced view.

The shafts 4, 5 can be supported at one end by, for example, bearings 13 or the like, whereby the shafts 4, 5 can rotate but their position will not be substantially changed by the weight caused by the sample 30.

Further, the device 1 comprises illuminating means 14 for illuminating the sample 30 from two or more directions. These illuminating means 14 can be, for example, light emitting diodes (LEDs) or other light emitting components. For illuminating the sample from two or more directions, illuminating means are provided in either two or more locations in connection with the device 1, and/or two or more illuminating beams are formed by means of mirrors, or the like. One aim of the illumination from different directions is to produce different shadows on the surface of the sample 30, whereby the roughness on the surface of the sample 30, such as pores, can be made more visible.

The device 1 is also equipped with imaging means 15 for imaging the sample 30. The imaging means 15 advantageously comprise one or more cameras, for example one, two or three cameras, which are placed so that the sample 30 can be imaged from a viewing angle that is suitable with respect to the illumination direction. One camera can be placed, for example, around the midpoint of the sample, to image the sample from above. In the case of using more than one camera, a second camera can be arranged, for example, to image the sample obliquely from the direction of one of its butt ends. Other alternatives for placing the camera/cameras and viewing angles are feasible as well. The imaging can comprise taking single images when moving the sample, whereby images can be taken of different parts of the sample 30, and the final image or images can be formed by combining the single images in a suitable way; and/or video recording, whereby a video image of the sample 30 is obtained when moving the sample.

In an advantageous embodiment of the invention, it is possible to use a so-called line scan camera, whereby the camera forms one row of pixels each scanning time. Thus, the final image is obtained by compiling all the pixel rows to one image after a sufficient number of images has been taken of the whole circumferential surface of the sample 30.

The images taken by the imaging means 15 can be transferred to analyzing means 16, in which various analyses can be made on the basis of the images, to find out properties of the sample. The analyzing means 16 can have various algorithms for different analyzing purposes.

Further, the device 1 comprises control means 17 for controlling the operation of the different parts of the device 1, such as moving, illuminating and imaging the sample 30. The control means 17 comprise, for example, a processor 18 and a memory 19, a computer, programmable logic circuitry, or the like. Moreover, the device 1 can be equipped with connecting means 20 (I/O, Input/Output) for converting signals generated by the control means 17 to signal levels which are suitable for the components to be controlled by the control means 17. In a corresponding manner, the connecting means 20 can be used to convert the signals intended to be read by the control means 17, to a format suitable for the control means 17.

In this context, it should be mentioned that the analyzing means 17 and the control means 17 can also be implemented in connection with the same processor 18 or computer, whereby the control commands intended to be executed by the processor 18 can be used to implement the desired analyzing and control functions. In this description, however, these various functions are called means, even if they were implemented primarily by software.

Moreover, the device 1 can comprise a user interface 21, such as a display and a keyboard, for controlling the operation of the device and for displaying the analysis results and the images taken by the imaging means. On the other hand, the user interface does not need to be a part of the device 1 according to the invention, but these can be implemented separately, or the display and/or the keyboard of the computer can be used for implementing the user interface.

In the following, we will describe the operation of the device 1 according to an advantageous embodiment of the invention in connection with analyzing a sample 30. In this example, the sample 30 used is a cylindrical concrete sample which is a cylinder removed from an existing concrete structure by, for example, diamond drilling. The diameter of the concrete sample is, for example, about 50 mm, but it may vary e.g. depending on the drilling device; the range is, for example, about 45 to 55 mm. The length of the sample will vary according to the structure to be examined, but in some cases it may be in the order of about 50 to 250 mm; it can also be thicker or thinner (for example 75 mm or 20 mm). The sample is washed, if needed, and for analyzing carbonation it is treated with e.g. a phenolphtalein indicator to determine the borderline between carbonated and uncarbonated concrete; the indicator will change its colour at a pH level of approximately 9. Uncarbonated concrete is more alkaline than this, and the pH of carbonated concrete is below 9.

The sample 30 is supported by rollers 4a, 5a; 4b, 5b, as illustrated in Figs. 1 and 2. The means 3 for moving the sample can be started, whereby the cylindrical concrete sample is rotated preferably at a constant speed. During the rotating, the sample is illuminated from different directions by illuminating means 14, and imaged by imaging means 15. These measures can be taken, for example, by first illuminating the sample from one direction and imaging it during one or more rotations or a partial rotation, followed by illuminating the sample from another direction and imaging it again during one or more rotations or a partial rotation. If necessary, further imagings can be performed by illuminating the sample 30 from other possible illumination directions as well. However, it is obvious that in some cases it will suffice to image the sample from one direction or two different directions. For detecting the surface profile, two or more different illumination directions may be needed, whereas for detecting differences in the surface colour, one illumination direction may be sufficient; or if the setting of the device is otherwise provided with sufficient lighting for imaging, a separate light source may not be needed.

In some embodiments, it is possible to use light sources of different colours, or light sources by which it is possible to generate light of different colours. It may also be expedient to use infrared (IR) light. The use of different colours and/or infrared may facilitate the detection of some properties of the sample.

In connection with the invention, it is also possible to apply other wavelengths, such as X-rays, ultrasound, or the like, for example to detect properties that cause such changes in the radiation reflected or emitted from the sample that can be detected by a detector suitable for the purpose. For example, if means emitting X-rays are used as the illuminating means 14, the imaging means 15 used can be such a detector or detectors which react to X-rays and convert the signals received by them to a format that can be transmitted to the analyzing means 16 for analysis. In some applications, the received X-rays can also be used to form an image that can be displayed on the display of the device. Yet another possibility to be mentioned is radiation of the object on the basis of magnetism, and the detection of possible changes caused by the radiation in the properties of the object. The invention can also be applied by applying a combination of two or more different forms of radiation, either simultaneously or in a sequence of time.

In some cases, the emission is focused on a part of the surface of the sample only, and the change in the properties of the sample on the basis of this radiation can be detected from said part as well. Thus, there may be a need to perform an analysis by rotating the sample several rotations and by moving the illuminating means 14 and/or the imaging means 15 in the longitudinal direction of the sample 30 so that substantially the whole outer surface of the sample can be analyzed.

For example, ultrasound can be directed to a sector of the surface of the sample, and the reflecting ultrasound can be received by *e.g.* an ultrasound receiver placed in the vicinity of the ultrasound source. The sample can first be rotated one rotation, to examine one part of the circumferential surface, after which the illuminating means 14 and/or the imaging means 15 are moved in the longitudinal direction of the sample 30 and the sample 30 is rotated again, for example one rotation. These steps can be repeated as many times as are needed.

It is obvious that particularly when using the above mentioned radiation wavelengths, the term illuminating means 14 can also refer to other than light emitting means. Correspondingly, the term imaging means 15 can also refer to other detectors than those reacting to visible light. In connection with the invention, it is also possible to use a combination of two or more different wavelengths or wavelength ranges, wherein the illuminating means 14 can comprise two or more different radiation sources, such as visible and infrared light. Correspondingly, the imaging means 15 can also consist of two or more detectors that react to different wavelengths or wavelength ranges.

A chemical treatment of the sample 30 is not necessarily needed if any of the radiation alternatives used leads to a change in the properties of the sample correspondingly. For example, if the reacting of the surface of the sample to X-rays depends on the acidity of the surface of the sample, it may be possible to find out e.g. the carbonation depth without having to cause colour changes according to the acidity on the surface of the sample.

Control means 17 can be used for controlling e.g. the operation of the imaging means 15. The control means 17 can, for example, control one camera to capture an image from a viewing angle from which the sample can be imaged in its whole width and length. Such an image can be transmitted to the analyzing means 16 in which the image can be used to determine the length and the diameter of the sample. This can be done, for example, by detecting the contours of the sample in the image. After determining the length and the diameter, it is possible to calculate the volume and the circumference of the cylindrical sample 30. When the circumference is known, it is also possible to determine how many rotations of the motor shaft will be needed for rotating the cylindrical sample 30 one rotation about its longitudinal axis. This information, in turn, can be used for controlling the imaging means to image the surface of revolution of the sample. If the surface of revolution is imaged several times (several rotations), for example with different lightings, it is thus possible to determine corresponding points in the different images. In other words, the images can be synchronized so that a given pixel represents the same point of the sample in the different images.

After imaging the sample 30 under different lightings a required number of times, the images can be analyzed by the analyzing means 16 to find out the desired properties. For example, the thickness of the surface layer (e.g. exposed aggregate concrete, concrete slab, brick tile, or clinker) can be determined by image processing methods. The carbonation depths may also be found out. Because e.g. the carbonation depth and/or the thickness of the surface layer is not necessarily constant on the whole circumference of the sample, it may also be advantageous to determine the minimum, the maximum, and the average of the carbonation depth and/or the surface layer thickness. Other properties to be possibly determined by using the device 1 include reinforcing steels (such as their number and diameter) and their location in the sample, rust scale, cracking, degree of compaction (= size, number and distribution of large pores, for example on a scale from 1 to 5), size of aggregate, type of cracking (such as plastic / dry shrinkage / structural, *etc.*).

As stated above in this description, the location and number of pores and other surface formations can be determined at some accuracy by utilizing shadows produced by illumination on the surface of the sample. By examining these shadows in different images, it is possible to determine the locations of the pores and also to estimate their size. After examining the whole circumferential surface, it is possible to determine e.g. the total number of pores on the surface of the sample. On the basis of this, it is thus possible to evaluate the effect of the pores on the structure from which the sample was taken. Figure 4 illustrates, by a recess 22 in the surface, how different shadows are formed in the recess by different lightings 23, 24. By examining and comparing the location, size and possible other visual properties of these shadows, it is possible to determine the size and the location of the deviation in the surface.

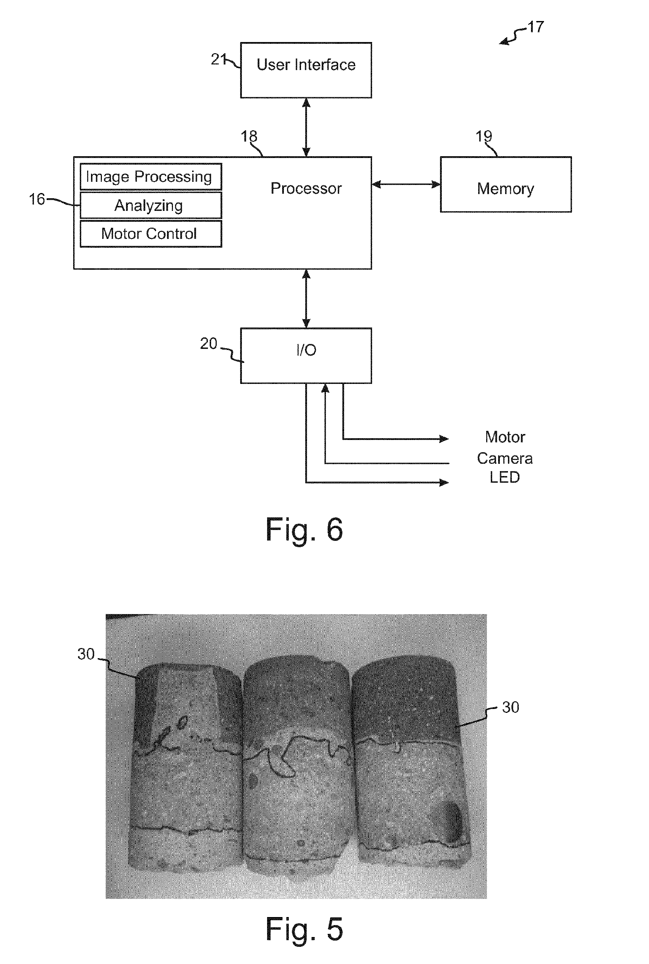

Figure 5 shows some examples of samples 30 in which colour changes caused by chemical treatment are visible. On the samples 30, some borderlines of colour changes have been highlighted by drawing by hand, but this is not always necessary when using the device 1; when analyzing the images of the samples 30, the resolution of the device 1 can be adjusted, if necessary, by means of the functional parameters of the device 1.

The analysis results determined by the analyzing means 16 can be displayed so that the user of the device 1 can draw the necessary conclusions from them.

Thus, the device 1 according to the invention can be used to implement the analysis of the samples 30 in a highly automated way, whereby human factors affect the analysis results to a lesser degree than when using solutions of prior art.

It will be obvious that the present invention is not limited solely to the above-presented embodiments but it can be modified within the scope of the appended claims.

## Claims

1. A device for analyzing a sample (30), the device (1) comprising at least:
- means (15) for imaging the sample (30), and
- means (14) for illuminating the sample (30),
**characterized in that** the device (1) also comprises
- means (3) for moving the sample (30) in at least one direction, and
- means (16) for analyzing the imaging information of the sample (30), for determining at least one property of the sample (30),
wherein said means (14) for illuminating the sample (30) are configured to illuminate the sample (30) from at least two different directions at different times, and said means (15) for imaging the sample (30) are configured to image the sample (30) when the sample (30) is being illuminated from said at least two different directions of illumination.

2. The device according to claim 1, **characterized in that** the means (3) for moving the sample are configured to rotate the sample about the longitudinal axis of the sample.

3. The device according to claim 2, **characterized in that** the means (3) for moving the sample comprise a first rotation shaft (4) configured to generate a force effect on the circumferential surface of the sample (30), for rotating the sample (30).

4. The device according to claim 2 or 3, **characterized in that** the means (3) for moving the sample (30) further comprise a second rotation shaft (5) configured to support the sample (30) at a point different from the point of support formed by said first rotation shaft (4) on the circumferential surface of the sample (30).

5. The device according to any of the claims 1 to 4, **characterized in that** the means (15) for imaging the sample (30) are configured to image the whole circumferential surface of the sample (30).

6. The device according to claim 5, **characterized in that** the means (16) for analyzing the imaging information of the sample (30) are configured to analyze the image information formed of the whole circumferential surface of the sample (30).

7. The device according to any of the claims 1 to 6, **characterized in that** the means (16) for analyzing the imaging information of the sample (30) are configured to analyze at least one of the following:
- porosity of the sample (30),
- number of pores in the surface of the sample (30),
- number of cracks in the surface of the sample (30),
- carbonation depth.

8. A method for analyzing a sample (30), the method comprising
- imaging the sample (30) and
- illuminating the sample (30),
**characterized in that** the method further comprises
- moving the sample in at least one direction, and
- analyzing the imaging information of the sample for determining at least one property of the sample,
wherein the method comprises illuminating the sample (30) from at least two different directions at different times, and imaging the sample (30) when the sample (30) is being illuminated from said at least two different directions of illumination.

9. The method according to claim 8, **characterized in** rotating the sample (30) about the longitudinal axis of the sample (30).

10. The method according to claim 9, **characterized in** generating a force effect on the circumferential surface of the sample (30) by a first rotation shaft (4), for rotating the sample.

11. The method according to claim 9 or 10, **characterized in** supporting the sample (30) by a second rotation shaft (5) at a point different from the point of support formed by said first rotation shaft (4) on the circumferential surface of the sample (30).

12. The method according to any of the claims 8 to 11, **characterized in** imaging the whole circumferential surface of the sample (30).

13. The method according to claim 12, **characterized in** analyzing the image information formed of the whole circumferential surface of the sample.

14. The method according to any of the claims 8 to 13, **characterized in** analyzing at least one of the following properties of the sample (30):
- porosity of the sample (30),
- number of pores on the surface of the sample (30),
- number of cracks on the surface of the sample (30),
- carbonation depth.
